# EUROPÄISCHE PATENTSCHRIFT

(11) **EP 1 371 346 B1**
(45) Veröffentlichungstag und Bekanntmachung des Hinweises auf die Patenterteilung: **13.02.2008**
(21) Anmeldenummer: 02013153.8
(22) Anmeldetag: 14.06.2002
(51) Int. Cl.: A61F 2/30, A61F 2/38, A61F 2/28, A61B 17/72

(54) **Implantierbare Prothese**
Implantable prosthesis
Prothèse implantable

(43) Veröffentlichungstag der Anmeldung: 17.12.2003
(73) Patentinhaber: Baumgart, Rainer, Dipl.-Ing. Dr. med., D-81479 München (DE)
(72) Erfinder: Baumgart, Rainer, Dipl.-Ing. Dr. med., D-81479 München (DE)
(74) Vertreter: Finck, Dieter

(56) Entgegenhaltungen:
- EP-A- 0 040 884
- EP-A- 0 729 732
- EP-A- 1 033 112
- EP-B- 0 432 253
- WO-A-00/30569
- WO-A-02/05732
- DE-A- 2 910 627
- DE-A- 19 633 865
- DE-A- 19 708 279
- DE-A- 19 906 423

## Beschreibung

Die Erfindung betrifft eine implantierbare Prothese zum Ersatz des menschlichen Hüft- oder Kniegelenks und der benachbarten Knochenschaftanteile, wobei die Prothese ein Gelenkteil, einen Schaftverankerungsteil und einen zwischen dem Gelenkteil und dem Schaftverankerungsteil angeordneten Schaftersatzteil aufweist.

Bei solchen im Handel befindlichen implantierbaren Prothesen (z.B. implantcast Feinguss GmbH, Buxtehude) kann der Schaftersatzteil verschiedene Längen aufweisen oder modular aufgebaut sein. Der Schaftverankerungsteil kann verschiedene Querschnitte haben, damit er den unterschiedlichen anatomischen Markraumdimensionen angepasst werden kann. Der Schaftverankerungsteil kann dabei eine Oberfläche, die ein festes Einwachsen in den Knochen ermöglicht, aufweisen.

Eine ähnlich aufgebaute gattungsgemäße implantierbare Prothese ist aus der WO 02/05732 A1 bekannt.

Künstliche Hüft- und Kniegelenke sind weit verbreitet und werden bei unterschiedlichen Indikationen eingesetzt. Wenn bei jüngeren Menschen Prothesen zum Einsatz kommen, kann es vorteilhaft sein, ein verkürztes Bein nach Implantation der Prothese zu verlängern. Insbesondere aber bei Kindern im Wachstumsalter, bei denen z.B. nach Entfernung eines Knochentumors Prothesen zum Einsatz kommen, bleibt das betroffene Bein regelmäßig im Wachstum zurück.

Bekannt sind Spezialprothesen (z.B. Fa. Stryker-Howmedica, Kiel), die eine Mechanik enthalten, mit der es möglich ist, den Prothesenkörper und damit das Bein zu verlängern. Nachteilig ist, dass der Verlängerungsvorgang z.B. durch Extrembewegungen unter Narkose aktiviert werden muss, der Verlängerungsbedarf meist akkumuliert auf große Intervalle appliziert wird und das Verhältnis von Prothese zu Restknochen immer größer wird.

Bekannt ist ferner ein vollimplantierbarer programmierbarer elektromotorischer Distraktionsmarknagel, in dem ein Elektromotor sowie ein Untersetzungsgetriebe angeordnet sind, mit dem über einen Spindelmechanismus Zugkräfte bis 1800 N realisiert werden können. Über ein Langloch oder einen Teleskopmechanismus und eine Bolzenschraube wird die Kraft auf den Knochen übertragen, was zu einer Distraktion im Osteotomiespalt (EP 0 432 253 B1) und zusätzlich bei geeigneter Distraktionsgeschwindigkeit zu einer Knochenneubildung führt.

Die Energieeinkopplung erfolgt durch Hochfrequenz über einen externen Sender, dem ein in einem Fenster im Marknagel angeordneter Empfänger zugeordnet ist (EP 1 033 112 A2) oder über einen auf die Haut aufgelegten Sender korrespondierend zu einer subkutan liegenden Empfangsantenne, die mit dem Motor in einem Nagelende über ein Kabel verbunden ist. Eine materielle Verbindung durch die Haut zu dem Implantat ist deshalb entbehrlich - es handelt sich also um ein vollständig implantierbares System (Der Orthopäde, Springer Verlag 1999, Band 28, Heft 12, Seite 1058 bis 1065).

Aus der DE 199 06 423 A1 ist ein aktiver Marknagel zur Distraktion von Knochensegmenten bekannt, mit dem ein Steckelement lösbar verbindbar ist, das über Verbindungsleitungen an eine andernorts deponierte Energieversorgung angeschlossen wird. Wenn der Marknagel als Hüft- oder Kniegelenksendoprothese verwendet wird, kann nach einer gewünschten Distraktion die elektrische Steckverbindung operativ herausgelöst oder, falls eine weitere Distraktion erforderlich wird, wieder eingesteckt werden.

Die der Erfindung zugrunde liegende Aufgabe besteht darin, die implantierbare Prothese der gattungsgemäßen Art so auszugestalten, dass mit ihr ohne Verbindung nach außen durch einen integrierten Antrieb der Knochen, in dem sie verankert ist, kontinuierlich verlängert und nach der Kallusdistraktionsmethode aufgebaut werden kann.

Diese Aufgabe wird ausgehend von der implantierbaren Prothese der gattungsgemäßen Art dadurch gelöst, dass der Schaftersatzteil und das künstliche Gelenk jeweils eine durchgehende zentrale Bohrung mit fluchtender Ausrichtung zueinander haben und dass der Schaftverankerungteil von einem Distraktionsmarknagel gebildet wird, der in die zentralen Bohrungen eingesetzt und im Gelenkteil und in einem gelenkfernen Knochenteil axial stabil, drehgesichert und lösbar befestigbar ist, und dass eine Befestigungseinrichtung für ein Verbinden eines durch Osteotomie gebildeten gelenknahen Knochenteils mit dem Schaftersatzteil vorgesehen ist.

Bei dieser Ausführungsform erfolgt eine Korrektur der bestehenden Längendifferenz zur Gegenseite durch Distraktion zwischen den verbliebenen, osteotomierten knöchernen Anteilen nach der Kallusdistraktionsmethode, so dass sich das Verhältnis von Prothese zu Knochen verkleinert.

Anstelle des Distraktionsmarknagels ist in die Bohrungen ein stabförmiger Stabilisator einsetzbar und im Gelenk und im gelenkfernen Knochenteil axial stabil, drehgesichert und lösbar befestigbar.

Dadurch ist es möglich, bei einem Kind nach Entfernen eines bösartigen Knochentumors, der auch das Entfernen des Gelenks erfordert, eine erfindungsgemäße Prothese zunächst mit dem Stabilisator als Schaftverankerungsteil zu implantieren, damit unmittelbar nach der Operation ein Ersatz für das fehlende Gelenk und den Knochen gegeben ist. Nach Abschluss der Chemotherapie, wenn sich der Organismus wieder erholt hat und in vollem Umfang wieder zur Knochenneubildung in der Lage ist, kann der Knochen in dem verbliebenen Bereich schonend durchtrennt und der Stabilisator in einem operativen Eingriff gegen einen Distraktionsmarknagel ausgetauscht werden. Nach abgeschlossener Distraktion kann der Distraktionsmarknagel wieder auf operativem Weg durch einen geeignet bemessenen Stabilisator ersetzt werden, der an seinem Schaftverankerungsteil jetzt eine Oberfläche aufweisen kann, die ein festes dauerhaftes Einwachsen im Knochen ermöglicht.

Um die axiale Kraftübertragung und die Sicherung gegen Verdrehung zu gewährleisten, werden die lösbaren Befestigungen von mit Formschluss quer durch das Gelenkteil und den Knochen sowie den Stabilisator bzw. den Distraktionsmarknagel verlaufenden Schraubenbolzen gebildet. Es ist aber auch möglich, axial zugängliche Verbindungselemente, z.B. Klemmschrauben, vorzusehen.

In weiterer Ausgestaltung der Erfindung erstreckt sich ein Kanal im Gelenkteil für eine Verbindung zwischen dem Antrieb und der Außenseite des Gelenks. Dies ermöglicht es, beispielsweise eine Empfangsantenne im Unterhautfettgewebe zu positionieren, damit sie dort von außerhalb des Gewebes über einen Sender Energie empfangen und über ein im Kanal angeordnetes Kabel dem Antrieb für seine Aktivierung zuführen kann. In dem Kanal kann auch ein Schlauch angeordnet werden, damit Aktivierungskomponenten im Subkutangewebe platziert und aktiviert werden können, beispielsweise Druck über ein Fluid auf eine Antriebskomponente innerhalb der Prothese übertragen werden kann.

Als Antrieb können ein elektromotorischer Antrieb, erforderlichenfalls mit einem zusätzliche Getriebe, ein Memorymetallantrieb, ein hydraulischer Antrieb, ein pneumatischer Antrieb oder ein piezoelektrischer Antrieb und dergleichen zum Einsatz kommen, der von außen her nach dem Prinzip Sender und Antenne oder durch Druckbeaufschlagung aktivierbar ist.

Die implantierbare Prothese nach der Erfindung eignet sich beispielsweise für die Positionierung am proximalen oder distalen Femur oder an der proximalen Tibia.

Anhand von Zeichnungen werden beispielsweise Ausführungsformen der Erfindung näher erläutert. Es zeigen:
- Fig. 1: schematisch im Schnitt eine erfindungsgemäße Prothese mit einem Distraktionsmarknagel nach einer Osteotomie,
- Fig. 2: in einer Ansicht wie Fig. 1 die Prothese mit dem Distraktionsmarknagel nach Distraktion und Kallusbildung,
- Fig. 3: in einer Ansicht wie Fig. 1 die Prothese mit einem Stabilisator, .

Die in den Zeichnungen gezeigte implantierbare Prothese hat einen Gelenkteil 10 mit einem Schaftersatzteil 12 und einem Schaftverankerungsteil 11, der in den Markraum eines Knochenteils 18 eingesetzt ist.

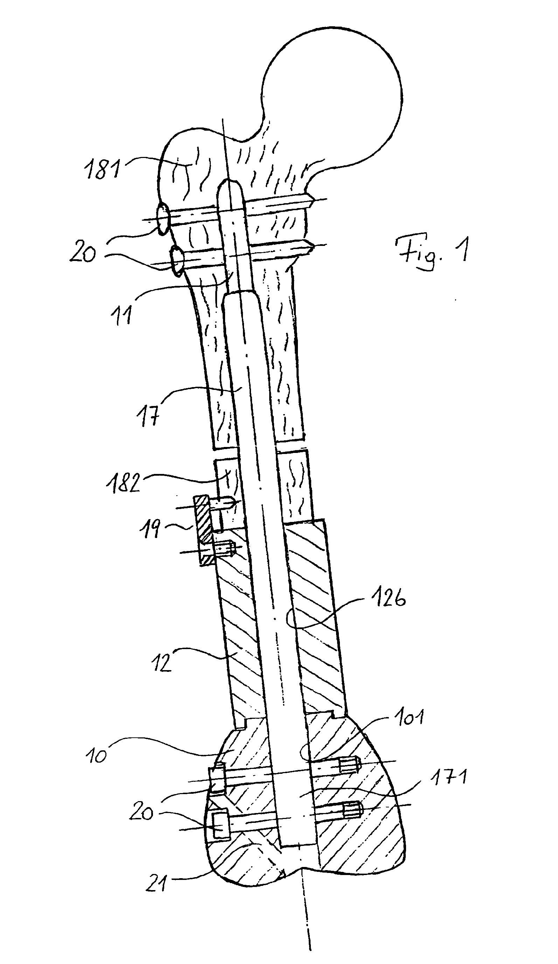

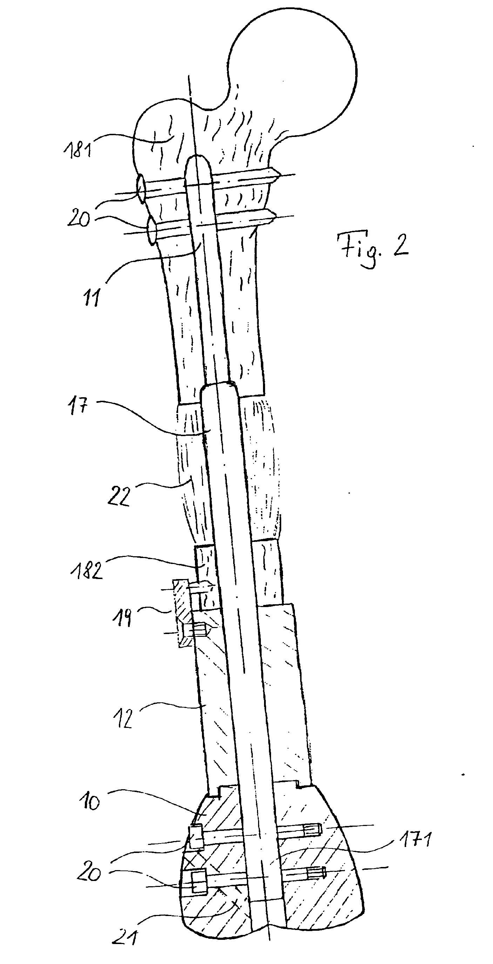

Bei der in Fig. 1 und 2 gezeigten Ausführungsform hat die implantierbare Prothese einen Schaftersatzteil 12 und ein Gelenkteil 10, durch die jeweils eine zentrale Bohrung 126, 101 hindurchgehen. Die zentralen Bohrungen 101 und 126 sind fluchtend zueinander ausgerichtet. Der Schaftersatzteil 12 ist an dem Gelenkteil 10 durch nicht gezeigte Befestigungsmittel, z.B. Gewinde- oder Hohlschraube, festgelegt. In die zentralen Bohrungen 101 und 126 ist ein Distraktionsmarknagel 17 eingesetzt, der, wie beispielsweise in der EP 0 432 253 B1 beschrieben ist, einen Hülsenteil 171 und einen daraus durch einen nicht gezeigten Antrieb ausfahrbaren Schaftverankerungsteil 11 aufweist. Der Distraktionsmarknagel 17 ist an einem gelenkfernen Knochenteil 181 mit seinem ausfahrbaren Schaftverankerungsteil 11 und am Gelenkteil 10 mit seinem Hülsenteil 171 durch quer verlaufende Schraubenbolzen 20 fixiert. Durch Osteotomie ergibt sich ein gelenknaher Knochenteil 182, der durch eine Befestigungseinrichtung 19 am Schaftersatzteil 12 festgelegt ist. Durch Aktivierung des im Distraktionsmarknagel 17 vorgesehenen Antriebs wird der Abstand zwischen dem gelenkfernen Knochenteil 181 und dem gelenknahen Knochenteil 182 in vorgeschriebenen Weise durch den Marknagel 17 langsam vergrößert, wobei durch die Bildung des Kallus 22 der natürliche Knochen allmählich verlängert und das Längenverhältnis von Prothese zu Knochen verringert wird.

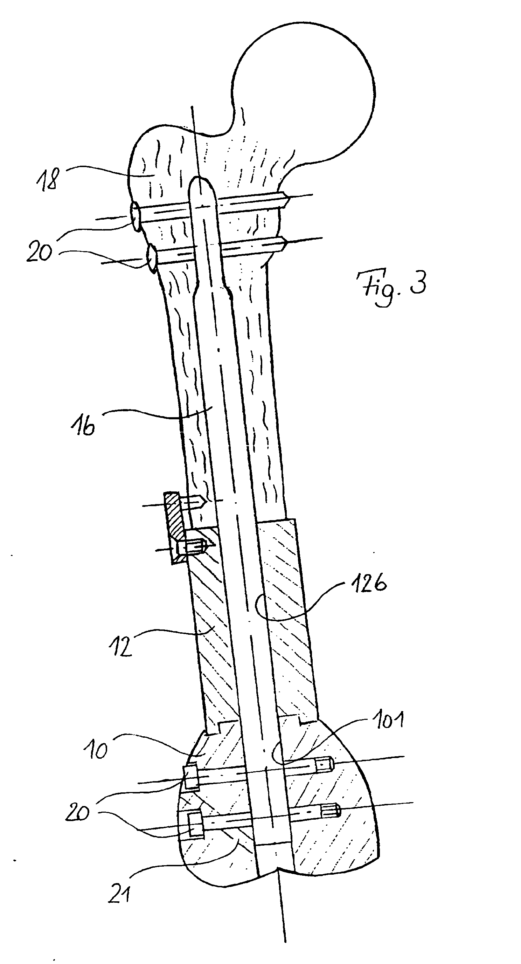

Nach Abschluss der Distraktion kann der Distraktionsmarknagel 17 (Fig. 2) durch einen entsprechend bemessenen stabförmigen Stabilisator 16 (Fig. 3) operativ ausgetauscht werden. Der Stabilisator 16 kann jedoch auch nach einer Tumoroperation während der Ausheilung lösbar eingesetzt und später für eine Distraktion operativ durch den in Fig. 1 und 2 gezeigten Distraktionsmarknagel 17 ausgetauscht werden. Gemäß Fig. 3 übernimmt der Stabilisator die Funktion des Schaftverankerungsteils. Der Stabilisator 16 ist im Knochen 18 und dem Gelenkteil 10 durch quer verlaufenden Schraubenbolzen 20 lösbar fixiert.

In den Ausführungsformen der implantierbaren Prothese von Fig. 1 und 2 ist im Gelenkteil 10 jeweils ein Kanal 21 für die Durchführung eines Kabels oder Schlauchs vorgesehen, wodurch eine Verbindung zwischen dem Antrieb 13 und der Außenseite des Gelenkteils 10 hergestellt wird, die den Anschluss des Schlauchs oder Kabels an einen subkutanen Empfänger für Druck oder HF-Energie ermöglicht.

## Patentansprüche

1. Implantierbare Prothese zum Ersatz des menschlichen Hüft- oder Kniegelenks und der angrenzenden Knochenschaftanteile, wobei die Prothese
- einen Gelenkteil (10),
- einen an dem Gelenkteil (10) befestigbaren Schaftersatzteil (12) und
- einen Schaftverankerungsteil aufweist, der in einem gelenkfernen Knochenteil (181) axial stabil und drehgesichert befestigbar ist,
**dadurch gekennzeichnet,**
- **dass** ein durch Osteotomie gebildeter gelenknaher Knochenteil (182) mit dem Schaftersatzteil (12) durch eine Befestigungseinrichtung (19) verbunden ist und
- **dass** der Schaftverankerungsteil ein Distraktionsmarknagel (17) ist, der sich von einer axial stabilen, drehgesicherten und lösbaren Befestigung (20) mit dem Gelenkteil (10) aus durch eine zentrale Bohrung (101) in diesem, eine fluchtend dazu ausgerichtete zentrale Bohrung (126) in dem Schaftersatzteil (12) und durch das gelenknahe Knochenteil (182) bis zu seiner lösbar ausgebildeten Befestigung (20) im gelenkfernen Knochenteil (181) erstreckt,
- wobei der Distraktionsmarknagel (17) aufgrund seiner lösbaren Befestigungen (20) durch einen ebenfalls axial stabil, drehgesichert und lösbar befestigbaren Stabilisator (16) austauschbar ist.

2. Implantierbare Prothese nach Anspruch 1, **dadurch gekennzeichnet, dass** die drehgesicherten lösbaren Befestigungen von mit Formschluss quer durch den Gelenkteil (10) und die Knochenteile (181, 182) sowie den Stabilisator (16) bzw. den Distraktionsmarknagel (17) verlaufenden Schraubenbolzen (20) gebildet werden.

3. Implantierbare Prothese nach Anspruch 1 oder 2, **gekennzeichnet durch** einen Kanal (21) im Gelenkteil (10) für eine Verbindung zwischen dem Antrieb (13) und der Außenseite des Gelenkteils (10).

## Claims

1. An implantable prosthesis for replacing the human hip or knee joint and the adjoining bone shaft portions, wherein the prosthesis,
- has a joint member (10),
- a shaft replacement part (12) which can be fastened to the joint member (10) and
- a shaft anchoring member which can be fastened in a manner which is axially stable and precludes relative rotation in a bone member (181) remote from the joint,
**characterised in,**
- **that** a bone member (182) near the joint and formed by osteotomy is connected to the shaft replacement part (12) by a fastening device (19) and
- **that** the shaft anchoring member is a distraction intramedullary pin (17) which extends from an axially stable, non-rotatable and releasable fastening (20) to the joint member (10) through a central bore (101) therein and through a central bore (126) aligned flush therewith in the shaft replacement part (12) and through the bone member (182) near the joint up to its releasably formed fastening (20) in the bone member (181) remote from the joint,
- wherein owing to its releasable fastenings (20) the distraction intramedullary pin (17) can be replaced by a stabiliser (16) which can also be releasably fastened in a manner which is axially stable and precludes relative rotation.

2. An implantable prosthesis according to Claim 1, **characterised in that** the non-rotatable releasable fastenings are formed by bolts (20) extending transversely through the joint member (10) and the bone members (181,182) with form-locking, as well as through the stabiliser (16) or the distraction intramedullary pin (17).

3. An implantable prosthesis according to Claim 1 or 2, **characterised by** a passage (21) in the joint member (10) for a connection between the drive (13) and the outside of the joint member (10).

## Revendications

1. Prothèse implantable pour le remplacement de l'articulation de la hanche ou du genou de l'homme et des parties de tige d'os contiguës, la prothèse présentant
- une partie articulée (10),
- une partie de remplacement de tige (12) pouvant être fixée sur la partie articulée (10) et
- une partie d'ancrage de tige, qui peut être fixée dans une partie d'os (181) éloignée de l'articulation axialement stable et bloquée contre la rotation,
**caractérisée en ce que**
- une partie d'os (182) proche de l'articulation et formée par ostéotomie est reliée à la partie de remplacement de tige (12) par un dispositif de fixation (19) et
- **en ce que** la partie d'ancrage de tige est un clou centro-médullaire de distraction (17), qui s'étend à partir d'une fixation (20) axialement stable, bloquée en rotation et amovible avec la partie articulée (10) à travers un perçage (101) central dans cette partie, un perçage (126) central orienté en alignement avec le premier perçage, dans la partie de remplacement de tige (12) et à travers la partie d'os (182) proche de l'articulation jusqu'à sa fixation (20) réalisée de façon amovible dans la partie d'os (181) éloignée de l'articulation,
- le clou centro-médullaire (17) pouvant être remplacé du fait de ses fixations (20) amovibles par un stabilisateur (16) également axialement stable, bloqué en orientation et fixé de façon amovible.

2. Prothèse implantable selon la revendication 1, **caractérisée en ce que** les fixations amovibles et bloquées en rotation sont formées par des boulons filetés agencés avec une complémentarité de forme transversalement à travers la partie articulée (10) et les parties d'os (181, 182) ainsi qu'à travers le stabilisateur (16) respectivement le clou centro-médullaire (17).

3. Prothèse implantable selon la revendication 1 ou 2, **caractérisée par** un canal (21) dans la partie d'articulation (10) pour une liaison entre l'entraînement (13) et le côté extérieur de la partie d'articulation (10).
